# EUROPÄISCHE PATENTANMELDUNG

(11) **EP 1 798 263 A2**
(43) Veröffentlichungstag der Anmeldung: **20.06.2007**
(21) Anmeldenummer: 06123304.5
(22) Anmeldetag: 01.11.2006
(51) Int. Cl.: C09C 1/00

(54) **Zinkoxid-Ceroxid-Kompositpartikel**

(30) Priorität: 13.12.2005 DE 102005059405
(71) Anmelder: Degussa GmbH, 40474 Düsseldorf (DE)
(72) Erfinder: Kröll, Michael, 63571, Gelnhausen (DE); Katusic, Stipan, 65779, Kelkheim (DE); Zimmermann, Guido, 63517, Rodenbach (DE); Heberer, Stefan, 63571, Gelnhausen-Meerholz (DE)

(57) **Zusammenfassung**

Kompositpartikel mit einer BET-Oberfläche von 5 bis 100 m²/g enthaltend eine Zinkoxid-Matrix und Ceroxid-Domänen, wobei sich die Domänen in der und auf der Matrix befinden und der Anteil an Zinkoxid 80 bis 98 Gew.-% und der Anteil an Ceroxid 2 bis 20 Gew.-%, jeweils bezogen auf die Kompositpartikel, beträgt.

## Beschreibung

Die Erfindung betrifft Zinkoxid-Ceroxid-Kompositpartikel, deren Herstellung und Verwendung.

Es ist bekannt, die photokatalytische Aktivität von anorganischen UV-Filtern, wie Titandioxid (UV-B) und Zinkoxid (UV-A), durch Umhüllen mit einer photokatalytisch inaktiven Komponente zu reduzieren.

In US 6132743 werden Zinkoxidpartikel offenbart, deren photokatalytische Aktivität durch Beschichtung mit einer Silikonverbindung reduziert wird.

US 6500415 offenbart Titandioxid- oder Zinkoxidpartikel, die mit Siliciumdioxid oder Aluminiumoxid umhüllt sind, um deren photokatalytische Aktivität zu minimieren.

In WO 03/037994 werden beispielsweise Titandioxidpartikel offenbart, deren photokatalytische Aktivität durch Umhüllung mit einem Oxid, Hydroxid oder eines Oxidhydroxides von Aluminium, Cer, Zirkon und/oder Silicium reduziert werden kann. Bei dem in WO 03/037994 offenbarten Verfahren wird eine Vorstufe des umhüllenden Materials mittels eines enzymatischen Fällungsmittelsystemes auf Titandioxidpartikel aufgebracht. Die umhüllten Partikel sind nicht aggregiert und weisen eine mittlere Partikelgröße von weniger als 50 nm auf. Als wesentlich für eine merkliche Reduzierung der photokatalytischen Aktivität ist eine vollständige Belegung notwendig.

Zusammenfassend offenbart der Stand der Technik UV-B-Filter, deren photokatalytische Aktivität durch Umhüllung mit einer photokatalytisch inaktiven Komponente, reduziert werden kann. Mit der Umhüllung wird in der Regel die UV-Absorption der umhüllten Substanz verringert, im besten Fall bleibt sie gleich. Die Wellenlänge der UV-Absorption wird durch die im Stand der Technik genannten umhüllenden Stoffe nicht oder nur gering verändert. Der Hülle kommt also lediglich die Aufgabe zu, die photokatalytische Aktivität zu minimieren, ohne die Absorption des UV-Filters im UV-B-Bereich zu sehr zu beeinträchtigen.

Die der vorliegenden Erfindung zugrunde liegende Aufgabe, besteht darin, einen UV-Filter mit nur geringer oder keiner photokatalytischen Aktivität bereitzustellen, der im UV-B- und UV-A-Bereich absorbiert.

Gegenstand der Erfindung sind Kompositpartikel mit einer BET-Oberfläche von 5 bis 100 m²/g, enthaltend eine Zinkoxid-Matrix und Ceroxid-Domänen, wobei sich die Domänen in der und auf der Matrix befinden und der Anteil an Zinkoxid 80 bis 98 Gew.-% und der Anteil an Ceroxid 2 bis 20 Gew.-%, jeweils bezogen auf die Kompositpartikel, beträgt.

Die erfindungsgemäßen Kompositpartikel können in aggregierter Form oder als isolierte Einzelpartikel vorliegen. Aggregierte Kompositpartikel weisen eine hohe Extinktion im UV-A und UV-B-Bereich auf und zeigen eine geringe photokatalytische Aktivität. Daher liegen die erfindungsgemäßen Kompositpartikel bevorzugt in aggregierter Form vor.

Die erfindungsgemäßen Partikel weisen einen Zinkoxid-Anteil von 80 bis 98 Gew.-% und einen Ceroxid-Anteil von 2 bis 20 Gew.-% auf. Bei einem Anteil von weniger als 2 Gew.-% ist noch keine nennenswerte Reduktion der photokatalytischen Aktivität im Vergleich zu reinem Zinkoxidpartikeln feststellbar. Bei Anteilen von Ceroxid von mehr als 20 Gew.-% ist keine weitere Reduzierung der photokatalytischen Aktivität mehr feststellbar.

Bevorzugterweise beträgt der Zinkoxid-Anteil 85 bis 95 Gew.-% und der Ceroxid-Anteil 5 bis 15 Gew.-%.

In den erfindungsgemäßen Kompositpartikeln beträgt die Summe der Anteile von Zinkoxid und Ceroxid vorzugsweise mindestens 99,9 Gew.-%, bezogen auf die Kompositpartikel. Insbesondere kann es vorteilhaft sein den Anteil an metallischen Verunreinigungen zu reduzieren. So können Kompositpartikel besonders vorteilhaft sein, dessen Anteile an Blei weniger als 20 ppm, an Arsen weniger als 3 ppm, an Cadmium weniger als 15 ppm, an Quecksilber weniger als 1 ppm, an Eisen weniger als 200 ppm und an Antimon weniger als 1 ppm, jeweils bezogen auf die Kompositpartikel, betragen.

Die BET-Oberfläche der Kompositpartikel beträgt vorzugsweise 20 bis 50 m²/g. In diesem Bereich weisen die Kompositpartikel hohe UV-A- und UV-B Absorption bei niedriger Photoaktivität auf.

Die Matrix der Kompositpartikel weist vorzugsweise einen mittleren Durchmesser von 20 bis 100 nm auf. In diesem Bereich weisen die Kompositpartikel hohe UV-A- und UV-B Absorption bei niedriger Photoaktivität auf.

Die Domänen der Kompositpartikel weisen vorzugsweise einen mittleren Durchmesser von 2 bis 10 nm auf. In diesem Bereich weisen die Kompositpartikel hohe UV-A- und UV-B Absorption bei niedriger Photoaktivität auf.

Ein weiterer Gegenstand der Erfindung ist ein Verfahren, bei dem man
- eine Lösung 1, welche eine oxidierbare Zinkverbindung enthält und eine Lösung 2, welche eine oxidierbare Cerverbindung enthält, mit einem Zerstäubungsgas mittels einer Düse in einen Reaktionsraum unter Bildung von Tropfen mit einem mittleren Durchmesser von weniger als 100 µm zerstäubt, wobei
- die Lösungsmittel vorzugsweise organischer Natur sind,
- der Anteil der Lösung 1 80 bis 98 Gew.-%, berechnet als ZnO, und der Anteil der Lösung 2 2 bis 20 Gew.-%, berechnet als CeO₂, beträgt,
- Lösung 1 eine Viskosität von 200 bis 5000 mPas und Lösung 2 eine Viskosität von 5 bis 150, vorzugsweise 10 bis 50 mPas, aufweist, und die Viskosität gegebenfalls durch Temperieren auf Temperaturen jeweils unterhalb der Zersetzungstemperatur der Zinkverbindung und der Cerverbindung geregelt wird,
- die zerstäubten Lösungen in eine Hochtemperaturzone eines Reaktors einbringt und dort bei Temperaturen von 800 bis 1200°C mit Sauerstoff oder einem sauerstoffhaltigen Gas zur Reaktion bringt,
- die heißen Gase und das feste Produkt kühlt und anschließend das feste Produkt von den Gasen abtrennt.

Vorzugsweise wird die in der Hochtemperaturzone benötigte Temperatur mit einer Flamme erzeugt, die aus der Reaktion eines wasserstoffhaltigen Brenngases mit Sauerstoff und/oder Luft erhalten wird. Die Temperatur kann durch das Verhältnis von wasserstoffhaltigem Brenngas und Sauerstoff eingestellt werden. Geeignete wasserstoffhaltige Brenngase können sein: Wasserstoff, Methan, Ethan, Propan, Butan und/oder Erdgas. Bevorzugt wird Wasserstoff eingesetzt.

Ein weiterer Gegenstand der Erfindung ist eine Dispersion, welche die erfindungsgemäßen Kompositpartikel enthält. Der Gehalt an Kompositpartikeln kann 0,1 bis 60 Gew.-%, bevorzugt von 10 bis 40 Gew.-%, bezogen auf die Gesamtmenge der Dispersion, betragen. Sie kann entweder wässerig oder organisch sein, oder aus einer Mischung bestehen, die Wasser und organische Lösungsmittel als flüssige Phase aufweist, wobei in allen Fällen nur eine einzige flüssige Phase vorliegt. Unter wässerig ist zu verstehen, dass der überwiegende Anteil der flüssigen Phase aus Wasser besteht. Unter organisch ist zu verstehen, dass die flüssige Phase überwiegend oder ausschließlich aus wenigstens einem organischen Lösungsmittel besteht. Geeignete organische Lösungsmittel können mono-, di-, tri-, poly-Alkohole, Ether, Ester, Aromaten, Alkane und Alkene sein. Insbesondere können Ethanol, Methanol, Propanol, Butanol, Aceton, Ethylacetat, Butylacetat eingesetzt werden. Das organische Lösungsmittel kann auch ein Reaktivverdünner, wie beispielsweise Hexandioldiacrylat oder Tripropylenglykoldiacrylat, sein.

Die erfindungsgemäße Dispersion kann weiterhin Additive enthalten. Dies können ein Dispergierhilfsmittel, ein Emulgator, ein pH-Wert regulierender Stoff und/oder ein Stabilisator sein. Vorzugsweise kann dies Na-Polyphosphat, Ascorbinsäure, Zitronensäure, 6-Aminohexansäure, Stearinsäure und/oder Salze der Polyacrylsäure, insbesondere das Natrium-Salz, sein. Weiterhin können Disperbyk 163, Disperbyk 180, Disperbyk 190 und/oder Byk 9077 eingesetzt werden. Das Additiv liegt vorzugsweise in einer Menge von 0,1 bis 5 Gew.-%, besonders bevorzugt von 0,5 bis 1,5 Gew.-%, bezogen auf die flüssige Phase der Dispersion, vor.

Ein weiterer Gegenstand der Erfindung ist eine Beschichtungszubereitung, welche die erfindungsgemäßen Kompositpartikel oder die erfindungsgemäße Dispersion und wenigstens ein Bindemittel enthält.

Geeignete Bindemittel können Polyacrylate, Polyurethane, Polyalkyde, Polyepoxide, Polysiloxane, Polyacrylonitrile und/oder Polyester sein. Bei Dispersionen, die einen oder mehrere Reaktivverdünner als flüssige Phase aufweisen, kann als Bindemittel ein aliphatisches Urethanacrylat, beispielsweise Laromer® LR8987, BASF, besonders geeignet sein. Besonders bevorzugt kann die erfindungsgemäße Beschichtungszubereitung Polyacrylate und/oder Polyurethane enthalten.

Der Anteil des Bindemittels in der Beschichtungszubereitung liegt bevorzugt zwischen 0,1 und 50 Gew.-%. Besonders bevorzugt ist ein Bereich zwischen 1 und 10 Gew.-%.

Der Anteil an Kompositpartikeln in der Beschichtungszubereitung liegt bevorzugt zwischen 0,1 und 60 Gew.-%. Besonders bevorzugt ist ein Bereich zwischen 1 und 10 Gew.-%.

Weiterhin kann die Beschichtungszubereitung während des Auftragens Verbindungen zur Veränderung der Rheologie der Beschichtungszubereitung enthalten. Besonders vorteilhaft sind Siliciumdioxid enthaltende Füllstoffe, wobei pyrogen hergestelltes Siliciumdioxid besonders bevorzugt ist. Die Menge kann bevorzugt zwischen 0,1 und 20 Gew.-%, bezogen auf die gesamte Beschichtungszubereitung, liegen.

Weiterhin kann die Beschichtungszubereitung organische Lösungsmittel wie Ethanol, Butylacetat, Ethylacetat, Aceton, Butanol, THF, Alkane oder Mischungen aus zwei oder mehreren dieser genannten Stoffe in Mengen von 1 Gew.-% bis 98 Gew.-%, bezogen auf die gesamte Beschichtungszubereitung, enthalten.

Die erfindungsgemäße Beschichtungszubereitung kann zur Beschichtung von Substraten aus Holz, PVC, Plastik, Stahl, Aluminium, Zink, Kupfer, MDF, Glas, Beton verwendet werden.

Ein weiterer Gegenstand der Erfindung ist eine Sonnenschutzformulierung, welche die erfindungsgemäßen Kompositpartikel enthält.

Der Anteil an Kompositpartikeln kann vorzugsweise 0,01 bis 25 Gew.-%, bezogen auf die Sonnenschutzformulierung, betragen. Daneben kann das erfindungsgemäße Sonnenschutzmittel in Mischungen mit bekannten anorganischen UV-absorbierenden Pigmenten und/oder chemischen UV-Filtern eingesetzt werden. Als bekannte UV-absorbierende Pigmente kommen in Betracht Titandioxide, Zinkoxide, Aluminiumoxide, Eisenoxide, Siliziumdioxid, Silicate, Ceroxide, Zirkoniumoxide, Bariumsulfat oder Gemische davon in Betracht. Als chemische UV-Filter kommen alle dem Fachmann bekannten wasser- oder öllöslichen UVA- als auch UV-B-Filter in Frage, von denen exemplarisch, jedoch nicht limitierend Sulfonsäurederivate von Benzophenonen und Benzimidazolen, Derivate des Dibenzoylmethans, Benzylidencampher und dessen Derivate, Derivate der Zimtsäure und deren Ester, oder Ester der Salizylsäure genannt seien. Die erfindungsgemäßen Sonnenschutzmittel können ferner die dem Fachmann bekannten Lösungsmittel wie Wasser, ein- oder mehrwertige Alkohole, kosmetische Öle, Emulgatoren, Stabilisatoren, Konsistenzregler wie Carbomere, Cellulosederivate, Xanthan-Gum, Wachse, Bentone, pyrogene Kieselsäuren und weitere in Kosmetika übliche Stoffe wie Vitamine, Antioxidantien, Konservierungsstoffe, Farbstoffe und Parfums enthalten.

Typischerweise kann das erfindungsgemäße Sonnenschutzmittel als Emulsion (O/W, W/O oder multipel), wässeriges oder wässerig-alkoholisches Gel oder Ölgel vorliegen, und in Form von Lotionen, Cremes, Milchsprays, Mousse, als Stift oder in anderen gebräuchlichen Formen angeboten werden.

Ein weiterer Gegenstand der Erfindung ist die Verwendung der erfindungsgemäßen Kompositpartikel, der erfindungsgemäßen Dispersion, der erfindungsgemäßen Beschichtungszusammensetzung oder der erfindungsgemäßen Sonnenschutzformulierung als UV-Filter.

### Beispiele

### Eingesetzte Materialien

Lösung A: Octa-Soligen Zink 10 (Fa. Borchers):
   Viskosität (20°C): 325 mPas
   Zinkoctoat: 10 Gew.-%, berechnet als Zink
   Testbenzin: 42 Gew.-%
   C₆-C₁₉-Fettsäuren: 46 Gew.-%
   2-(2-Butoxyethoxyethanol): 2 Gew.-%
Lösung B: Borchi Kat 22, Fa. Borchers,
   Viskosität (20°C): 5017 mPas
   Zinkoctoat: 22 Gew.-%, berechnet als Zink
   C₆-C₁₉-Fettsäuren: 78 Gew.-%
Lösung C: Octa-Soligen Cerium 12, Fa. Borchers, Viskosität (30°C): 61 mPas
   Ceriumoctoat 50 Gew.-%
   2-Ethylhexansäure 50 Gew.-%.
Lösung D: Cer(III)nitrat-hexahydrat gelöst in Aceton, Gehalt an Cer 8 Gew.-%, Viskosität (20°C): 52 mPas

Beispiel 1: 1463 g/h Lösung A mit einer Temperatur von 20°C, die bei dieser Temperatur eine Viskosität von 325 mPas aufweist und 136 g/h Lösung C mit einer Temperatur von 80°C , die bei dieser Temperatur eine Viskosität von 9,2 mPas aufweist werden mittels einer Dreistoffdüse in einen Reaktionsraum unter Bildung eines Aerosoles zerstäubt. Der mittlere Tropfendurchmesser D₃₀ beträgt weniger als 100 µm. Hier brennt eine Knallgasflamme aus Wasserstoff (3 Nm³/h) und Primärluft (5 Nm³/h), in der das Aerosol zur Reaktion gebracht wird. Zusätzlich wird Sekundärluft (25 Nm³/h) in den Reaktionsraum eingebracht. Die Temperatur 0,5 m unterhalb der Flamme beträgt 925°C. Nachfolgend wird das Reaktionsgemisch abgekühlt und das feste Produkt an einem Filter von gasförmigen Stoffen abgetrennt.

Die Beispiele 2 bis 6 werden analog durchgeführt. Einsatzstoffe und Einsatzmengen sind in Tabelle 1 wiedergegeben. Zusammensetzung und BET-Oberfläche der erhaltenen Kompositpartikel sind ebenfalls in Tabelle 1 wiedergegeben.

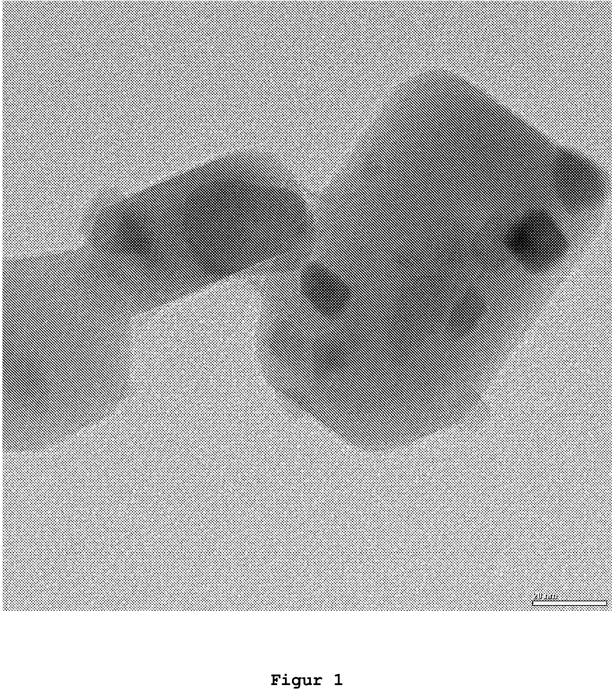

Figur 1 zeigt eine TEM-Aufnahme der Kompositpartikel aus Beispiel 2. Es sind deutlich die dunkleren Ceroxid-Domänen in einer Zinkoxidmatrix zu erkennen. Der Durchmesser der Zinkoxidmatrix beträgt im allgemeinen 20 bis 100 nm, der der Ceroxid-Domänen im Allgemeinen 5 bis 10 nm. Der Zinkoxid-Anteil liegt in hexagonaler, der Ceroxid-Anteil in kubischer Form vor.

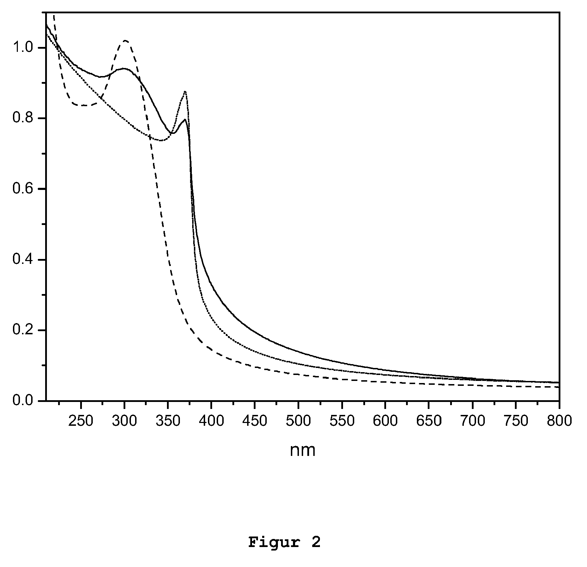

Figur 2 zeigt die Extinktion von ZnO (.........), CeO₂ (----) und den erfindungsgemäßen Kompositpartikeln aus Beispiel 2(------) in Abhängigkeit von der Wellenlänge in nm. Deutlich ist bei dem erfindungsgemäßen Kompositpartikeln, welche ca. 90 Gew.-% Zinkoxid und ca. 10 Gew.-% Ceroxid enthalten, zu erkennen, dass die Extinktion trotz des geringen Anteiles an Ceroxid nur geringfügig kleiner ist als bei reinem Ceroxid.

**Tabelle 1: Einsatzstoffe und Einsatzmengen; analytische Werte der erhaltenen Kompositpartikel**

| **Beispiel** | | **1** | **2** | **3** | **4** | **5** | **6** |
|---|---|---|---|---|---|---|---|
| Lösung A | | | | | | | |
| Konzentration | g/h | 1385 | 1463 | - | - | 1461 | 1853 |
| Temperatur | °C | 23 | 23 | - | - | 23 | 23 |
| Viskosität | mPas | 325 | 325 | - | - | 325 | 325 |

| Lösung B | | | | | | | |
|---|---|---|---|---|---|---|---|
| Konzentration | g/h | - | - | 1034 | 1057 | - | - |
| Temperatur | °C | - | - | 23 | 23 | - | - |
| Viskosität | mPas | - | - | 5000 | 5000 | - | - |

| Lösung C | | | | | | | |
|---|---|---|---|---|---|---|---|
| Konzentration | g/h | 115 | 136 | 166 | 343 | - | - |
| Temperatur | °C | 80 | 80 | 80 | 80 | - | - |
| Viskosität | mPas | 9,2 | 9,2 | 9,2 | 9,2 | - | - |

| Lösung D | | | | | | | |
|---|---|---|---|---|---|---|---|
| Konzentration | g/h | - | - | - | - | 139 | 47 |
| Temperatur | °C | - | - | - | - | 40 | 40 |
| Viskosität | mPas | - | - | - | - | 32 | 32 |
| Wasserstoff | Nm³/h | 3 | 3 | 2 | 2 | 3 | 5 |
| Verdüsungsluft | Nm³/h | 5 | 5 | 4 | 5 | 5 | 6 |
| Primärluft | Nm³/h | 25 | 25 | 20 | 20 | 25 | 20 |
| Sekundärluft | Nm³/h | 10 | 10 | 10 | 10 | 15 | 15 |
| Temperatur | °C | 950 | 925 | 900 | 900 | 975 | 1050 |
| ZnO | Gew.% | 91 | 90 | 92 | 85 | 93 | 98 |
| CeO₂ | Gew.% | 9 | 10 | 8 | 15 | 7 | 2 |
| BET-Oberfläche | m²/g | 28 | 36 | 30 | 45 | 26 | 25 |

Auf der anderen Seite reicht die Extinktion von Zinkoxidanteilen in den Kompositpartikeln nahezu an die Extinktion von reinem Zinkoxid heran. Es liegt also ein synergistischer Effekt dahingehend vor, dass die integrale Extinktion der Kompositpartikel größer ist als sich arithmetisch aus der Summe der einzelnen Komponenten (10% Ceroxid, 90 % Zinkoxid) ergeben würde. Die erfindungsgemäßen Kompositpartikel zeichnen sich weiterhin dadurch aus, dass deren Bestandteile im UV-A und UV-B-Bereich eine hohe Absorption aufweisen und somit als Breitbandfilter verwendet werden können.

### Beispiel 7: Photokatalytische Aktivität

Die photokatalytische Aktivität von Zinkoxid (Vergleich) und den Kompositpartikeln aus Beispiel 2 wird anhand des Abbaus von Dichloressigsäure (DCA) unter Verwendung eines auf 20°C thermostatisierten Bestrahlungsreaktors mit einem Quarzglasfenster von 4,9 cm² Fläche und einem Volumen von 250 ml durchgeführt. Bestrahlt wird mit einer 450 W Xenon-Lampe (Osram XBO) und einer Bestrahlungsstärke von 65 mW/cm². Die Bestrahlungsdauer beträgt mindestens 4 Stunden. Das zu testende Material liegt als 0,1 %-ige Dispersion in Wasser vor. Um die Ionenstärke während des gesamten Versuches konstant zu halten, werden der Dispersion zusätzlich 10 mM KNO₃ zugesetzt. Die Anfangskonzentration der Dichloressigsäure (DCA) betrug 1 mM. Der pH-Wert der kontinuierlich gerührten Dispersion wurde durch Zugabe von 0,1 M NaOH konstant gehalten, wobei 0,1 M NaOH zutitriert wurde.

Der Abbau der DCA kann unmittelbar anhand des Verbrauchs an Natronlauge zur Konstanthaltung des pH-Wertes verfolgt werden. Es gilt folgende Stöchiometrie:

CHCl₂COO⁻ + O₂ -> H⁺ + 2 Cl⁻ + 2 CO₂

Aus den Anfangssteigungen der erhaltenen Protonenbildungskurven kann die Abbaurate (nM/s) und daraus die auf die eingestrahlte Lichtintensität bezogene Photoneneffizienz (%) bestimmt werden.

Reines Zinkoxid ergibt hiermit eine DCA-Abbaurate von 21,5 nM/s und eine Photoneneffizienz von 0,58%. Die erfindungsgemäßen Kompositpartikel aus Beispiel 2 hingegen weisen eine DCA-Abbaurate von lediglich 4,23 nM/s und eine Photoneneffizienz von 0,11% auf.

Selbst bei einem Anteil von Ceroxid von nur 10 Gew.-% und der Tatsache, dass der Großteil der Oberfläche der eingesetzten Kompositpartikel Zinkoxid ist, kann die photokatalytische Aktivität deutlich reduziert werden. Es wurde weiterhin gefunden, dass die photokatalytische Aktivität durch Anteile an Ceroxid von mehr als 20 Gew.-% nicht mehr nennenswert reduziert werden kann.

### Beispiel 8: Herstellung einer erfindungsgemäßen Dispersion

Zu 50 g Wasser, dem 0,1 Gew.-% Polyacrylsäure in Form des Natriumsalzes zugesetzt sind, werden portionsweise unter Umrühren die Kompositpartikel aus Beispiel 2 gegeben, bis ein Festoffgehalt von 10 Gew.-% resultiert. Anschließend wird jeweils eine Minute mit einem Ultraschallfinger (Durchmesser: 7 mm, Gerät: Ultraschallprozessor UP 400s, Leistung: 400 W, Dr. Hielscher) dispergiert.

### Beispiel 9: Herstellung einer erfindungsgemäßen Beschichtungszubereitung auf Acryl-/Polyurethan-Basis

Die Dispersion aus Beispiel 8 wird unter dispergierenden Bedingungen zu einer handelsüblichen Acryl-/Polyurethan-Bindemittelzubereitung gegeben (Relius Aqua Siegel Gloss), so dass eine Beschichtungszubereitung mit einem Anteil an Kompositpartikeln von 2 Gew.-% resultiert.

### Beispiel 10: Herstellung einer erfindungsgemäßen Beschichtungszubereitung auf Acryl-Basis

Durchführung wie bei Beispiel 9, jedoch unter Verwendung einer handelsüblichen Acryl-Bindemittelzubereitung. (Macrynal SM 510 (Cytec), Desmodur N75 (Bayer).

### Beispiel 11: UV-Beständigkeit bei Beschichtung von Holz

Mit den Beschichtungszubereitungen aus Beispiel 9 und 10 werden je 3 Kiefernholz-Proben, die mit einem Primer (Relius Aqua Holz Grund) vorbehandelt wurden, beschichtet (QUV-B 313; DIN EN 927-6, ISO 11507, ASTM D 4857). Als Vergleich dienen Kiefernholz-Proben, die mit einer Beschichtungszubereitung, die frei von Kompositpartikeln ist, auf Acryl-/Polyurethan-Basis (Relius Aqua Siegel Gloss) beschichtet.

Nach einer Testzeit von 1000 Stunden zeigen die Beschichtungen aus den Beispielen 9 und 10 im Gegensatz zur Beschichtung ohne Kompositpartikel eine deutlich geringere Vergilbung, einen deutlichen höheren Glanz und keine Versprödung oder Risse in der Beschichtung.

### Beispiel 12: Härte bei Beschichtung von Glas

Die Beschichtungszubereitungen aus den Beispielen 9 und 10, sowie eine Beschichtungszubereitung ohne kompositpartikel auf Acryl-/Polyurethan-Basis (Relius Aqua Siegel Gloss) als Vergleich, werden in einer Schichtdicke von 150 µm auf Glasplatten aufgetragen. Die Härte wird nach Trocknungszeiten von 1, 6, 13 und 34 Tagen bei normalen Laborbedingungen (20°C, 65% RH) (DIN ISO 1522) bestimmt. Die Härte der Schichten hervorgegangen aus den Beispielen 9 und 10 ist bis zu 100% größer als die des Vergleichsbeispieles.

### Beispiel 13: Glanz und Weißgrad auf beschichteten Metallplatten

Die Metallplatten werden mit einem weißen Lack vorbehandelt. Anschließend werden die Beschichtungszusammensetzungen 9 und 10 und eine handelsübliche Beschichtungszusammensetzung mit einem organischen UV-Filter behandelt und 55 Tage gemäß DIN53231 bestrahlt.

Der Weißgrad nach Berger der Probe mit organischem UV-Filter ist nach dieser deutlich geringer als die Proben aus den Zusammensetzungen der Beispiele 9 und 10.

### Beispiel 14: Herstellung einer erfindungsgemäßen Sonnenschutzformulierung

Mit nachfolgender Rezeptur wurde ein Sonnenschutzmittel mit 4 Gew.-% der erfindungsgemäßen Partikel nach Beispiel 2 hergestellt.

| **Phase** | **Bestandteil** | **Gew.-%** |
|---|---|---|
| A | Isolan GI 34 | 3,0 |
| | Rizinusöl | 1,2 |
| | Tegesoft OP | 10,0 |
| | Tegesoft Liquid | 5,0 |
| | Glycerin 86% | 3,0 |
| B | Paracera W80 | 1,8 |
| | Isohexadecan | 5,0 |
| C | Kompositpartikel nach Beispiel 2 | 4,0 |
| D | Magnesiumsulfat | 0,5 |
| | VE-Wasser | 66,5 |

Phase A wird in einem Mischer auf 70°C erwärmt. Nach dem Aufschmelzen auf einer Magnetheizplatte bei 80°C wird Phase B zu Phase A gegeben. Die Phase C wird mit ca. 300 U/min und unter Vakuum in die Ölphase eingerührt. Phase D wird ebenfalls auf 70°C erwärmt und unter Vakuum der Mischung aus A-C zugefügt.

## Patentansprüche

1. Kompositpartikel mit einer BET-Oberfläche von 5 bis 100 m²/g enthaltend eine Zinkoxid-Matrix und Ceroxid-Domänen, wobei sich die Domänen in der und auf der Matrix befinden und der Anteil an Zinkoxid 80 bis 98 Gew.-% und der Anteil an Ceroxid 2 bis 20 Gew.-%, jeweils bezogen auf die Kompositpartikel, beträgt.

2. Kompositpartikel nach Anspruch 1, **dadurch gekennzeichnet, dass** sie in aggregierter Form vorliegen.

3. Kompositpartikel nach den Ansprüchen 1 oder 2, **dadurch gekennzeichnet, dass** der Zinkoxid-Anteil 85 bis 95 Gew.-% und der Ceroxid-Anteil 5 bis 15 Gew.-%, jeweils bezogen auf die Kompositpartikel, beträgt.

4. Kompositpartikel nach den Ansprüchen 1 bis 3, **dadurch gekennzeichnet, dass** der Anteil von Zinkoxid und Ceroxid mindestens 99,9 Gew.-%, bezogen auf die Kompositpartikel, beträgt.

5. Kompositpartikel nach den Ansprüchen 1 bis 4, **dadurch gekennzeichnet, dass** die BET-Oberfläche 20 bis 50 m²/g beträgt.

6. Kompositpartikel nach den Ansprüchen 1 bis 5, **dadurch gekennzeichnet, dass** die Matrix einen mittleren Durchmesser von 20 bis 100 nm aufweist.

7. Kompositpartikel nach den Ansprüchen 1 bis 6, **dadurch gekennzeichnet, dass** die Domänen einen mittleren Durchmesser von 2 bis 10 nm aufweisen.

8. Verfahren zur Herstellung der Kompositpartikel gemäß den Ansprüchen 1 bis 7, **dadurch gekennzeichnet, dass**
- man eine Lösung 1, welche eine oxidierbare Zinkverbindung enthält und eine Lösung 2, welche eine oxidierbare Cerverbindung enthält, mit einem Zerstäubungsgas mittels einer Düse in einen Reaktionsraum unter Bildung von Tropfen mit einem mittleren Durchmesser von weniger als 100 µm zerstäubt, wobei
- der Anteil der Lösung 1 80 bis 98 Gew.-%, berechnet als ZnO, und der Anteil der Lösung 2 2 bis 20 Gew.-%, berechnet als CeO₂, beträgt,
- Lösung 1 eine Viskosität von 200 bis 5000 mPas und Lösung 2 eine Viskosität von 5 bis 150 mPas, aufweist, und die Viskosität gegebenfalls durch Temperieren auf Temperaturen jeweils unterhalb der Zersetzungstemperatur der Zinkverbindung und der Cerverbindung geregelt wird,
- die zerstäubten Lösungen in eine Hochtemperaturzone eines Reaktors einbringt und dort bei Temperaturen von 800 bis 1200°C mit Sauerstoff oder einem sauerstoffhaltigen Gas zur Reaktion bringt,
- die heißen Gase und das feste Produkt kühlt und anschließend das feste Produkt von den Gasen abtrennt.

9. Verfahren nach Anspruch 9, **dadurch gekennzeichnet, dass** die Reaktionstemperatur durch eine Flamme erzeugt wird, die aus der Reaktion eines wasserstoffhaltigen Brenngases mit Sauerstoff und/oder Luft resultiert.

10. Dispersion enthaltend die Kompositpartikel gemäß der Ansprüche 1 bis 7.

11. Beschichtungszusammensetzung enthaltend die Kompositpartikel gemäß der Ansprüche 1 bis 7 oder die Dispersion gemäß des Anspruches 11 und wenigstens ein Bindemittel.

12. Sonnenschutzformulierung enthaltend die Kompositpartikel gemäß der Ansprüche 1 bis 7 oder die Dispersion gemäß des Anspruches 11.

13. Verwendung der aggregierten Kompositpartikel gemäß der Ansprüche 1 bis 7, der Dispersion gemäß des Anspruches 11, der Beschichtungszusammensetzung gemäß des Anspruches 12 oder der Sonnenschutzformulierung gemäß Anspruch 13 als UV-Filter.
